(19) Europäisches Patentamt
European Patent Office
Office européen des brevets

(11) EP 4 668 540 A2

## (12) EUROPEAN PATENT APPLICATION

(43) Date of publication:
24.12.2025 Bulletin 2025/52

(21) Application number: 25213841.7

(22) Date of filing: 29.11.2022

(51) International Patent Classification (IPC):
*H02J 50/12* (2016.01)

(52) Cooperative Patent Classification (CPC):
**A61M 60/178; A61M 60/216; A61M 60/873;
H02J 50/005; H02J 50/12;** A61M 2205/36;
A61M 2205/8243; H02J 2310/23

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR

(30) Priority: 01.12.2021 US 202163284780 P

(62) Document number(s) of the earlier application(s) in
accordance with Art. 76 EPC:
22823299.7 / 4 440 679

(71) Applicant: TC1 LLC
St. Paul, MN 55117 (US)

(72) Inventors:
• HANSEN, John Freddy
St. Paul (Minnesota), 55117 (US)
• BAVAL, Alexander
St. Paul (Minnesota), 55117 (US)

• HARJES, Daniel I.
St. Paul (Minnesota), 55117 (US)
• ANDERSON, Russell Eugene
St. Paul (Minnesota), 55117 (US)
• IUDICE, Jeff
St. Paul (Minnesota), 55117 (US)

(74) Representative: Deambrogi, Edgardo et al
Jacobacci & Partners S.p.A.
Corso Emilia 8
10152 Torino (IT)

Remarks:
This application was filed on 06.11.2025 as a
divisional application to the application mentioned
under INID code 62.

## (54) SYSTEMS AND METHODS FOR CONDUCTIVITY COATINGS ON WIRELESS POWER RESONATORS

(57) Resonators for use in a transcutaneous energy transfer system (TETS) are provided. A resonator includes a housing, and a magnetic core positioned within the housing, the magnetic core including an annular sidewall and a central post that define an annular groove. The resonator further includes a coil element positioned within the annular groove and surrounding the central post, and a metal object coated with a conductive material, wherein the conductive material facilitates reducing an amount of heat induced during operation of the resonator.

EP 4 668 540 A2

## Description

### CROSS-REFERENCE TO RELATED APPLICATIONS

This application claims priority to provisional application serial No. 63/284,780, filed December 1, 2021, which is incorporated herein by reference in its entirety.

### BACKGROUND OF THE DISCLOSURE

### Field of the Disclosure

The present disclosure generally relates to wireless power transfer systems, and more specifically, relates to wireless power transfer resonators including conductivity coatings.

### Background

Ventricular assist devices, known as VADs, are implantable blood pumps used for both short-term (i.e., days or months) and long-term (i.e., years or a lifetime) applications where a patient's heart is incapable of providing adequate circulation, commonly referred to as heart failure or congestive heart failure. A patient suffering from heart failure may use a VAD while awaiting a heart transplant or as a long-term destination therapy. In another example, a patient may use a VAD while recovering from heart surgery. Thus, a VAD can supplement a weak heart (i.e., partial support) or can effectively replace the heart's natural function.

A wireless power transfer system may be used to supply power to the VAD. However, in some cases, the wireless power transfer system may generate undesirable heat.

### SUMMARY OF THE DISCLOSURE

The present disclosure is directed to conductivity coatings on components of a resonator for use in a wireless power transfer system.

In one aspect, a resonator for use in a transcutaneous energy transfer system (TETS) is provided. The resonator includes a housing, a magnetic core positioned within the housing, the magnetic core including an annular sidewall and a central post that define an annular groove, a coil element positioned within the annular groove and surrounding the central post, and a metal object coated with a conductive material, wherein the conductive material facilitates reducing an amount of heat induced during operation of the resonator.

In another aspect, a wireless power transfer system is provided. The wireless power transfer system includes an external transmit resonator, and an implantable receive resonator, the implantable receive resonator including a housing, a magnetic core positioned within the housing, the magnetic core including an annular sidewall and a central post that define an annular groove, a coil element positioned within the annular groove and surrounding the central post, and a metal object coated with a conductive material, wherein the conductive material facilitates reducing an amount of heat induced during operation of the implantable receive resonator.

In yet another aspect, a method of assembling a resonator for use in a transcutaneous energy transfer system (TETS) is provided. The method includes positioning a magnetic core within a housing, the magnetic core including an annular sidewall and a central post that define an annular groove, positioning a coil element within the annular groove, and coating a metal object of the resonator with a conductive material, wherein the conductive material facilitates reducing an amount of heat induced during operation of the resonator.

### BRIEF DESCRIPTION OF THE DRAWINGS

FIG. 1 is a simplified electrical circuit diagram of a wireless power transfer system according to an example embodiment.

FIG. 2 is an illustration of the wireless power transfer system of FIG. 1 used to supply power to a ventricular assist device (VAD) according to an example embodiment.

FIG. 3 is a perspective view of one example of a resonator that may be used to implement the system shown in FIG. 1 according to an example embodiment.

FIG. 4 is a perspective, cross-sectional view of a resonator assembly that may be used to implement the system shown in FIG. 1 according to an example embodiment.

FIG. 5 is a perspective view of an example embodiment of a resonator that may be used to implement the system shown in FIG. 1 according to an example embodiment.

FIGs. 6A and 6B are thermal diagrams of the resonator shown in FIG. 5.

### DETAILED DESCRIPTION OF THE DISCLOSURE

As indicated above, a VAD may receive power from, or otherwise be powered by, a wireless power transfer system. Although a VAD is specifically mentioned, other implantable devices may be powered by wireless power transfer systems. In an example, the wireless power transfer system includes an external transmit resonator and an implantable receive resonator. The receive resonator is configured to be implanted inside a patient's body. The power transfer system may also be referred to as a transcutaneous energy transfer system (TETS).

A TETS operates by a transmitter coil generating an oscillating magnetic field which induces a voltage in a receiving coil. Although a coil is specifically mentioned, other structures (e.g., stacked and/or capacitively coupled plates) may be used to generate the voltage. One drawback to the TETS is the generation of undesirable voltages in nearby metal objects. These voltages are undesirable because they drive currents in the metal objects which, in turn, may generate undesirable heat. For example, an alternating current associated with or otherwise generated by the TETS may flow through an outer layer of each metal object or other such conductor (known as the "skin effect"). As the alternating current flows through the outer layer, heat is generated.

In order to address the above, the present application describes coating various metal objects in the TETS with a highly conductive material such as, for example, silver, copper, gold, and/or aluminum. Although silver, copper, gold, and aluminum are specifically mentioned, other coating materials may be used. Coating the metal objects with the highly conductive metal will help reduce or eliminate the amount of heat generated by mirror currents or image currents. Additionally, it may be possible to predict the location of where the currents will be concentrated. Once the location is determined, the conductive coating may be selectively applied to that area.

As used herein 'coat' or 'coating' does not necessarily mean that the material was applied in a coating process where molten or vaporized metal was applied to a surface and allowed to solidify. Rather, the coatings described herein may include a solid thin metal foil or strip or band that is placed over or around an underlying object, either partially or fully encircling it, or partially or fully wrapping it.

Accordingly, examples of the present disclosure describe systems and methods for conductivity coatings on wireless power transfer resonators. A resonator includes a housing, and a magnetic core positioned within the housing, the magnetic core including an annular sidewall and a central post that define an annular groove. The resonator further includes a coil element positioned within the annular groove and surrounding the central post, and a metal object coated with a conductive material, wherein the conductive material facilitates reducing an amount of heat induced during operation of the resonator.

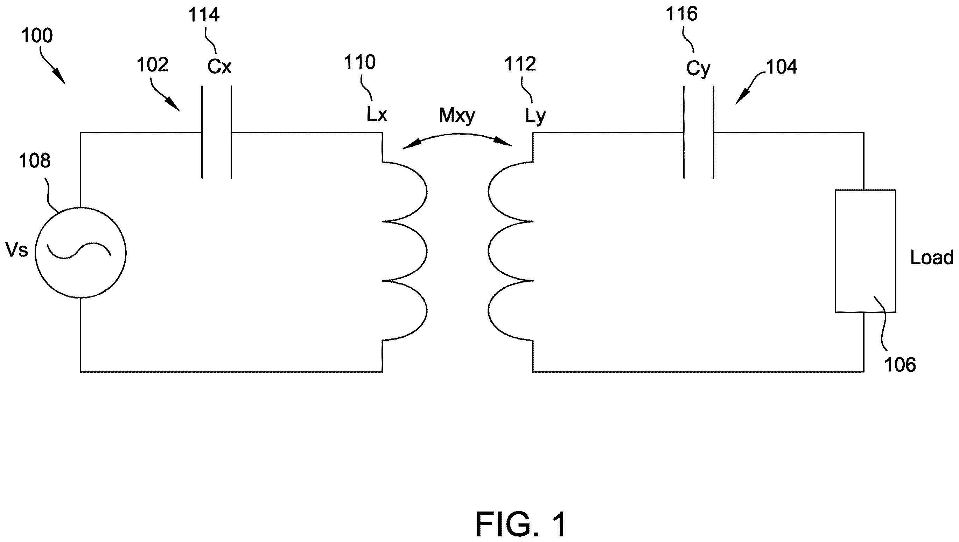

Referring now to the drawings, FIG. 1 illustrates a simplified circuit of a wireless power transfer system 100 according to an example embodiment. System 100 includes an external transmit resonator 102 and an implantable receive resonator 104. In the system shown in FIG. 1, a power source Vs 108 is electrically connected with the transmit resonator 102, thereby providing power to the transmit resonator 102. The receive resonator 104 is connected to a load 106 (e.g., an implantable medical device). The receive resonator 104 and the load 106 may be electrically connected with a switching or rectifying device (not shown).

In an example, the transmit resonator 102 includes a coil Lx 110 connected to the power source Vs 108 by a capacitor Cx 114. Further, the receive resonator 104 includes a coil Ly 112 connected to the load 106 by a capacitor Cy 116. Inductors Lx 110 and Ly 112 are coupled by a coupling coefficient k. $M_{xy}$ is the mutual inductance between the two coils. The mutual inductance, $M_{xy}$, is related to the coupling coefficient k as shown in the below Equation (1).

$$M_{xy} = k\sqrt{L_x \cdot L_y} \ (1)$$

In operation, the transmit resonator 102 transmits wireless power received from the power source Vs 108. Receive resonator 104 receives the power wirelessly transmitted by transmit resonator 102 and transmits the received power to load 106.

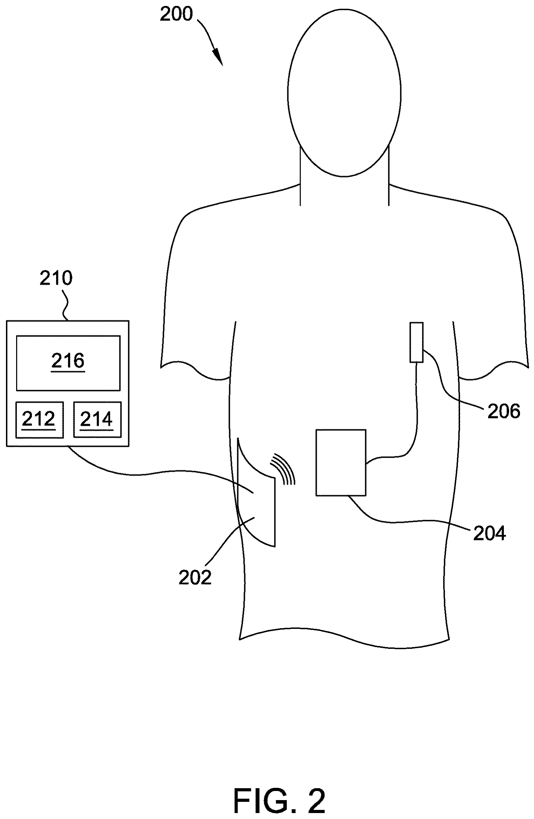

FIG. 2 illustrates an example of a patient 200 using an external coil 202 (e.g., a transmit resonator 102 (FIG. 1)) to wirelessly transmit power to an implanted coil 204 (e.g., a receive resonator 104 (FIG. 1)). Implanted coil 204 uses the received power to power an implanted device 206. For example, implanted device 206 may include a pacemaker or heart pump (e.g., a left ventricular assist device (LVAD)). In some examples, implanted coil 204 and/or implanted device 206 may include or be coupled to a battery.

In one example, external coil 202 is communicatively coupled to a computing device 210, for example, via wired or wireless connection, such that the external coil 202 may receive signals from and transmit signals to the computing device 210. In some examples, the computing device 210 is a power source for the external coil 202. In other examples, the external coil 202 is coupled to an alternative power supply (not shown). The computing device 210 includes a processor 212 in communication with a memory 214. In some examples, executable instructions are stored in the memory 214.

The computing device 210 further includes a user interface (UI) 216. The UI 216 presents information to a user (e.g., the patient 200). For example, the UI 216 may include a display adapter that may be coupled to a display device, such as a cathode ray tube (CRT), a liquid crystal display (LCD), an organic LED (OLED) display, and/or an electronic ink display. In some examples, the UI 216 includes one or more display devices. Further, in some examples, the UI may be or otherwise include a presentation interface. The presentation interface may not generate visual content, but may generate audible and/or computer-generated spoken-word content. In an example, the UI 216 displays one or more representations designed to aid the patient 200 in placing the external coil 202 such that the coupling between the external coil 202 and the implanted coil 204 is optimal. In some examples, the computing device 210 may be a wearable device such as, for example, a wristwatch.

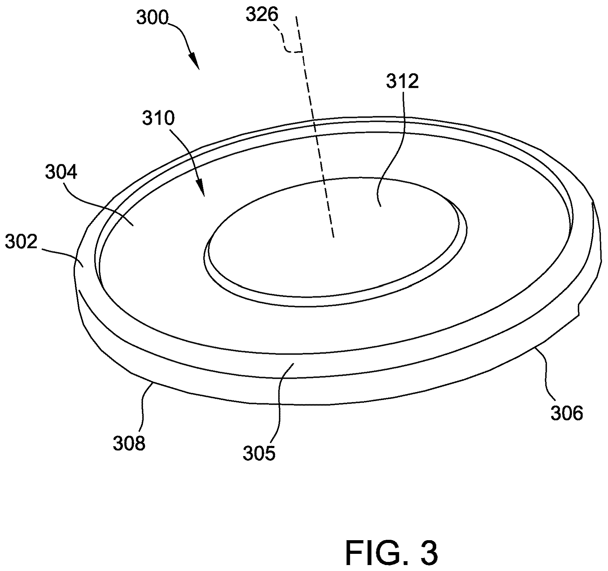

FIG. 3 is a front perspective view of one example of a resonator 300 that may be used to implement the system 100 shown in FIG. 1. For example, the resonator 300 may be used to implement the external transmit resonator 102 (FIG. 1), the implantable receive resonator 104 (FIG. 1), the external coil 202 (FIG. 2), and/or the implanted coil 204 (FIG. 2).

In an example, the resonator 300 includes a core 302 and a coil element 304. The core 302 includes a front surface 305, a back surface 306, and an annular sidewall 308 extending between the front surface 305 and the back surface 306. An annular groove 310 is defined by the front surface 305 and forms a central post 312 of the core 302.

The resonator 300 (including the core 302 and the coil element 304) functions as a wireless power resonator when coupled to a capacitor (e.g., a capacitor on a printed circuit board electrically coupled to coil element 304). However, those of skill in the art will appreciate that resonator 300, without connection to a capacitor, constitutes a coil assembly. Accordingly, as used herein, the term resonator does not require that the device be coupled to a capacitor to form a wireless power resonator. In contrast, as used herein, the term resonator is broad enough to cover a coil assembly that includes a core and a coil element without connection to a capacitor, as shown in FIG 3.

In an example, the core 302 is formed of a magnetic material. The magnetic material may be a ferrite material, such as nickel-based or manganese-based ferrites. Nickel-based ferrites generally have lower electrical conductivity and reduced losses, while manganese-based ferrites have a higher magnetic permeability (while still having acceptable losses), facilitating containing magnetic field lines, and reducing fringing fields entering nearby conductors (e.g., a titanium enclosure or copper in a nearby PCB) to prevent losses. In other examples, other types of ferrite materials may be used. For example, in some examples, a magnesium-based ferrite (e.g., MgCuZn, which may outperform nickel-based and manganese-based ferrites in a frequency range around 1 Megahertz (MHz)) may be used.

The coil element 304 is positioned within the annular groove 310 and surrounds the central post 312. The resonator 300 may be, for example, a Litz wire resonator or a stacked plate resonator. In a Litz wire resonator, the coil element 304 includes a plurality of loops of Litz wire. In a stacked plate resonator, the coil element 304 includes a plurality of stacked plates that may include a plurality of alternating dielectric layers and conductive layers arranged in a stack. The dielectric layers may be formed of, for example, ceramic, plastic, glass, and/or mica.

The coil element 304 may be electrically coupled to a power source (e.g., when functioning as a transmit resonator) or a load (e.g., when functioning as a receive resonator). In operation, when power is supplied to the resonator 300 operating as a transmit resonator, current flows through the coil element 304, creating an inductive current loop. This inductive current loop is capable of wirelessly transmitting power to a second resonator 300, provided that resonance frequencies of the first and second resonators 300 overlap. The coil element 304 may include a plurality of terminals (not shown) that facilitate electrically coupling the coil element 304 to a power supply or load.

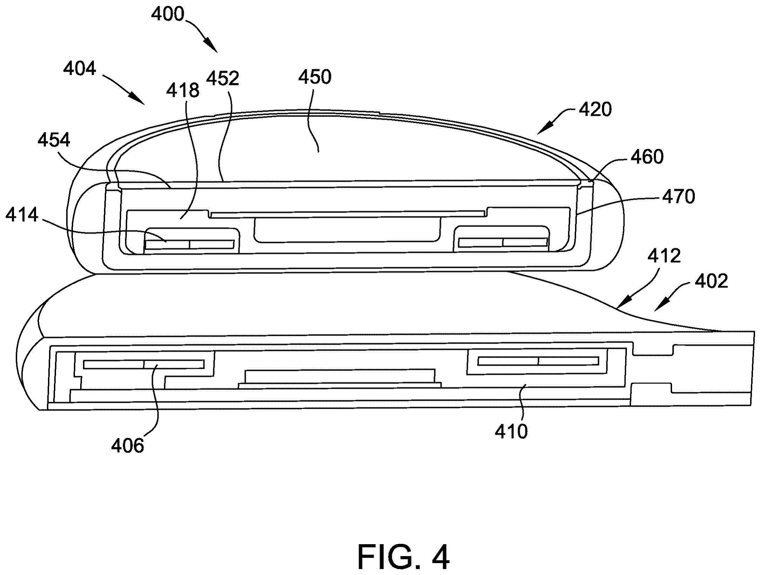

FIG. 4 is a perspective view of a resonator assembly 400. Resonator assembly 400 may include resonators similar to the resonator 300 shown in FIG. 3. The resonator assembly 400 includes a transmit resonator 402 and a receive resonator 404. In an example, the transmit resonator 402 includes a first coil element 406 and a first core 410 positioned within a first housing 412 (e.g., a ceramic housing). Similarly, the receive resonator 404 includes a second coil element 414 and a second core 418 positioned within a second housing 420 (e.g., a ceramic housing). As explained above, the receive resonator 404 is typically implanted within the body, while the transmit resonator 402 is typically external to the body. The transmit and receive resonators 402 and 404 may include one or more electronic components (not shown), such as field-effect transistors (FETs), series inductors, and/or other electronic components.

In an example, one or more metal objects in or on the transmit and receive resonators 402 and 404 are covered with a coat of a highly conductive material. As described above, the highly conductive material may be silver, copper, gold, and/or aluminum. Although silver, copper, gold, and aluminum are specifically mentioned, other materials may be used as the coating material. Notably, highly conductive metals (such as silver, copper, gold, and aluminum) promote high electric conductivity. In examples where highly conductive metals are not used, a metal object may be coated with a metal having a higher conductivity than that of the metal object being coated.

The currents generated by the transmit resonator 402 and the receive resonator 404 are alternating currents (ACs) in the example embodiment. The ACs may cause a "skin effect" to occur, the skin effect pushing some, most, or all of the ACs to or close to the surface of some or all of the coated metal objects. The skin effect may be present to some extent at all typical operating frequencies (e.g., 250 kHz, 1 MHz, 3.3 MHz, 6.78 MHz) of the resonator assembly 400. In some examples, the skin effect may become more significant at higher frequencies.

In some examples, the particular effects of the skin effect and/or where the ACs may be concentrated on the various electronic components may be predicted (e.g., by a human operator and/or a computing device). For example, a computing device may execute various simulations (e.g., finite element methods) to determine the effects of the skin effect on each electronic component.

Once the determination and/or the prediction of the locations(s) at which the ACs are or will be concentrated has been made, a highly conductive coating material may be applied to various portions the metal objects. As indicated above, the coating material may be silver, copper, gold, aluminum, and/or other suitable materials. The high conductivity coating material facilitates reducing heat that would otherwise be generated by the concentration of ACs at those locations.

In some examples, the entire surface of the metal object is coated. However, selectively coating portions of the metal object may be more cost effective.

In general, placement of the coating material should be carefully monitored and controlled. For example, careless placement of high conductivity coatings may reduce a magnetic coupling between the transmit resonator 402 and the receive resonator 404, reducing the efficiency of the wireless power arrangement. For example, placing coated electronic components in close proximity to one of the coils themselves, or in the region between the two coils, may reduce the magnetic coupling capabilities of the system.

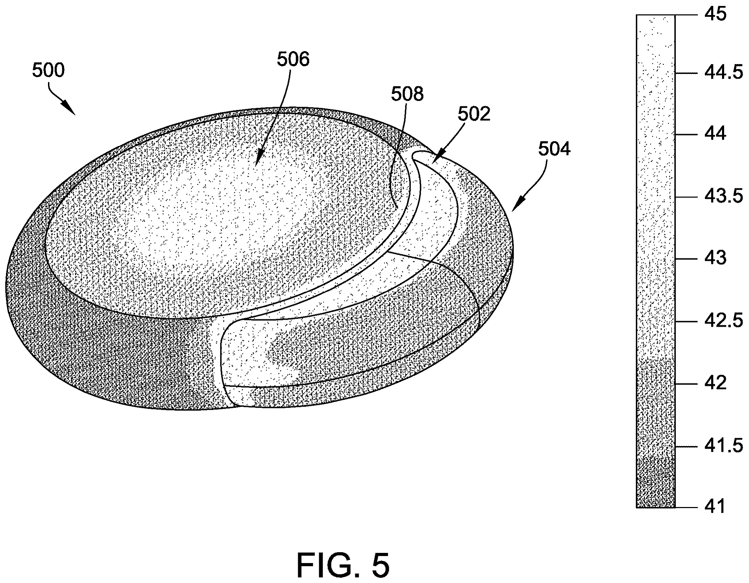

FIG. 5 is a perspective view of an implantable TETS resonator 500 according to an example embodiment. FIG. 5 also shows thermal information regarding the TETS resonator 500. The implantable TETS resonator 500 may be similar to the resonator 300 shown and described with respect to FIG. 3 and/or the receive resonator 404 shown and described with respect to FIG. 4. The TETS resonator 500 includes a bulge-shaped metal header block 502. The metal header block 502 may be positioned on the edge of the TETS resonator 500 and/or may be coupled to the TETS resonator 500. The metal header block 502 may include a substantially planar top surface with a rounded sidewall. In an example, the metal header block 502 may include an interior surface (not shown) facing the rest of TETS resonator 500. The rounded sidewall may be part of an exterior surface 504 that faces away from the TETS resonator 500.

In the example shown in FIG. 5, metal header block 502 includes titanium. Although titanium is discussed herein, other materials may be used. In this example, the heat induced in the metal header block 502 during operation of the TETS resonator 500 is approximately 0.5 W. As shown in FIG. 5, the highest temperatures on the TETS resonator 500 are found at or near a center 506 of the TETS resonator 500, and at or near an edge 508 of the TETS resonator 500 that is adjacent metal header block 502. High temperatures are also found on the surface of metal header block 502, including exterior surface 504.

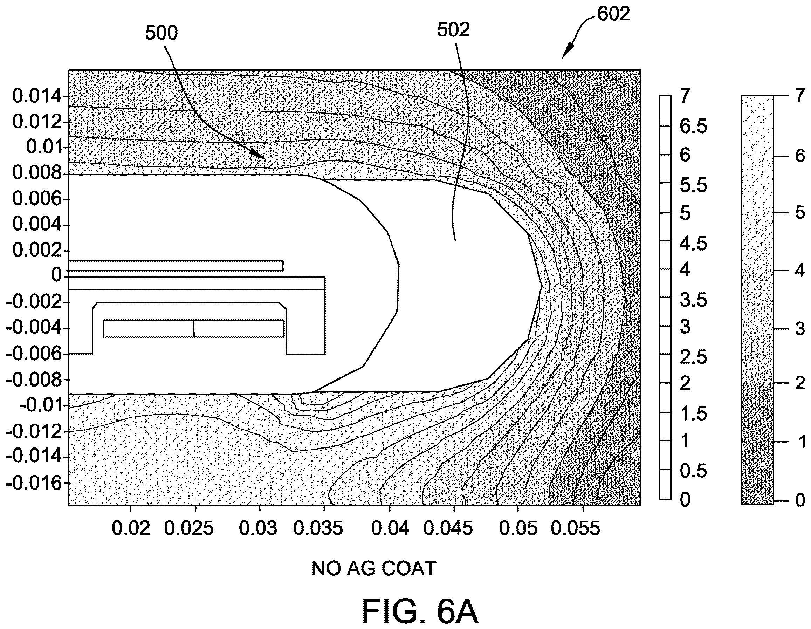

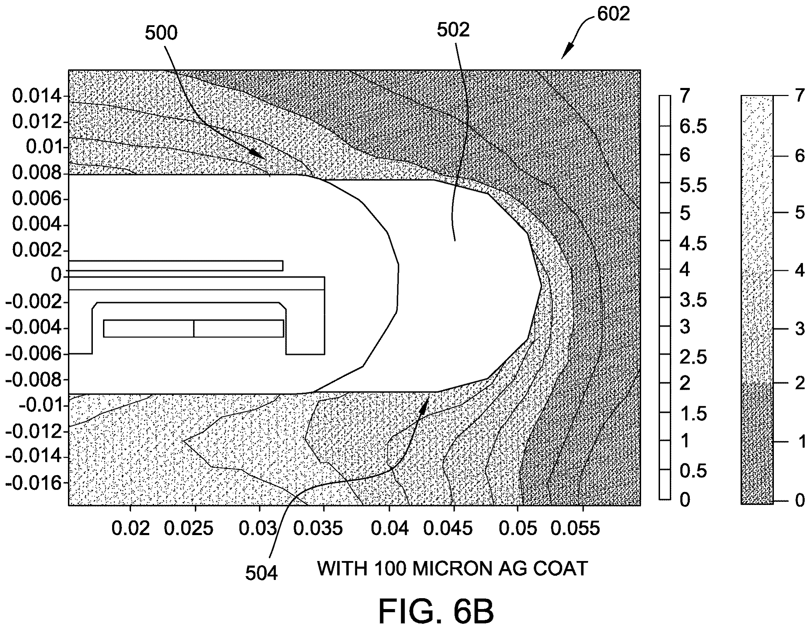

FIGs. 6A and 6B are cross-sectional thermal views, including a plurality of surface temperatures 602, of the implantable TETS resonator 500 (shown in FIG. 5). In FIG. 6A, the TETS resonator 500 includes the metal header block 502 made of titanium, and the metal header block 502 is not coated with a conductive coating. In contrast, in FIG. 6B, the metal header block 502 includes a coating of a highly conductive material on the exterior surface 504. In this example, the conductive coating is a silver coating with a thickness of about 100 microns ($\mu$m). Notably, by coating the exterior surface 610 with the conductive coating (as in FIG. 6A), the induced heat is greatly reduced.

For example, in the embodiment of FIG. 6A (without the coating), the heat induced by the header block 502 was approximately 0.5 Watts (W). In contrast, with the coating included, the heat induced by the header block 502 is approximately 0.028W, and the heat induced by the coating is approximately 0.060 W, resulting in total induced heat of 0.088W (as compared to 0.5W). While the added conductive coating does have its own Ohmic losses, the conductive coating shields the underlying titanium, reducing Ohmic loss in the underlying titanium. As a result, the amount of heat generated by the TETS resonator 600 is substantially reduced.

Referring back to FIG. 4, in this embodiment, the receive resonator 404 further includes a metal disk 450 on a side of the receive resonator 404 opposite the transmit resonator 402. The metal disk 450 may be fabricated from, for example, titanium. The metal disk 450 includes an exterior surface 452 and an interior surface 454 (i.e., that faces the second coil element 414). Without using a conductive coating, during operation of the receive resonator 404, approximately 0.5 W of heat may be induced within the metal disk 450. However, when a high conductivity coating (e.g., copper) is applied to the interior surface 454, the induced heat is reduced to approximately 0.084 W. Specifically, the induced heat for the metal disk 450 is 0.031 W, and the induced heat for the conductive coating is 0.053 W. The conductive coating functions as a heat spreader, distributing heat over the receiver resonator 404. Further, the conductive coating may coat all, or only a portion, or the interior surface 454.

In the embodiment of FIG. 4, the receive resonator 404 also includes a metal ring 460 that circumscribes the metal disk 450. The metal ring 460 may be fabricated from the same metal as the metal disk 450 (e.g., titanium). The metal ring 460 may be welded or otherwise coupled to the metal disk 450, and functions as an interface between the metal disk 450 and the second housing 420. Further, the metal ring 460 may be brazed to or otherwise coupled to the second housing 420.

In this example, the conductive coating on the interior surface 454 of the metal disk 450 does not facilitate shielding the metal ring 460 from magnetic fields. For example, the induced heat may be as follows: 4.9 milliwatts (mW) in the metal disk 450; 8.9 mW in the conductive coating; 180 mW in the metal ring 460; and 75.7 mW in a braze material between the metal ring 460 and the second housing 420 (resulting in a total induced heat of approximately 269.5 mW). However, the total amount of induced heat may be reduced in a several ways.

For example, the braze material may modified to reduce induced heat. In one embodiment, the braze material may be made from an alloy of gold, titanium, and/or other metals. Accordingly, the braze material may have an electric conductivity that is inferior to that of other materials (e.g., pure gold). Modifying the braze material to be fabricated from pure gold results in shielding the metal ring 460 and reducing induction heat loss by about two thirds, and result in a total heat loss of up to approximately 137.3 mW. However, this may impact the mechanical strength of the braze material (although this may be relatively unimportant in the implantable receive resonator 404).

In another example, a high conductivity coating (e.g., copper) may be applied to an interior side wall of the receive resonator 404 (e.g., sidewall 470). In this example, the coating may form a shape similar to a shallow cup. With a taller interior side wall (and thus more coating), the more the heat is reduced. For example, if the receive resonator 404 includes a 4 millimeter (mm) tall copper-coated sidewall, a total induced heat may be approximately 191.9 mW. However, in this example, the 4 mm tall copper-coated sidewall may interfere with the coupling between the transmit resonator 402 and the receive resonator 404. In another example, a 2 mm tall copper-coated sidewall may avoid interfering with the coupling between the transmit resonator 402 and the receive resonator 404, and may have a total induced heat of 209.9 mW. It should be noted that the techniques described above may be combined with one another. For example, combining a pure gold braze material with a 2 mm tall copper-coated sidewall may result in a total induced heat of approximately 134.7 mW.

In yet another aspect, a highly conductive coating may be applied to the outer diameter of the metal ring 460. For example, adding a silver coat to the outer diameter of the metal ring 460, and also using a pure gold braze material, may result in reducing the total induced heat to approximately 89.1 mW, and in another example to approximately 20 mW.

The examples described herein are directed to systems and methods for wireless power transfer resonators. A resonator includes a housing, and a magnetic core positioned within the housing, the magnetic core including an annular sidewall and a central post that define an annular groove. The resonator further includes a coil element positioned within the annular groove and surrounding the central post, and a metal object coated with a conductive material, wherein the conductive material facilitates reducing an amount of heat induced during operation of the resonator.

Although the examples and examples disclosed herein have been described with reference to particular examples, it is to be understood that these examples and examples are merely illustrative of the principles and applications of the present disclosure. It is therefore to be understood that numerous modifications can be made to the illustrative examples and examples and that other arrangements can be devised without departing from the spirit and scope of the present disclosure as defined by the claims. Thus, it is intended that the present application cover the modifications and variations of these examples and their equivalents.

This written description uses examples to disclose the disclosure, including the best mode, and also to enable any person skilled in the art to practice the disclosure, including making and using any devices or systems and performing any incorporated methods. The patentable scope of the disclosure is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

**Claims**

1. A resonator (400, 402) for use in a wireless power transfer system (200), the resonator comprising:

   a housing (412, 420);
   a magnetic core (410, 418) positioned within the housing, the magnetic core defining an annular groove (310); and
   a coil element (304) positioned within the annular groove,
   **characterized in that** the resonator further comprises a metal object, wherein a conductive material is applied to at least a portion of the metal object, wherein the conductive material facilitates reducing an amount of heat induced during operation of the resonator.

2. The resonator (400, 402) of claim 1, wherein the resonator is an implantable receive resonator (204).

3. The resonator (400, 402) of claim 1, wherein the conductive material is silver, copper, gold and/or aluminum.

4. The resonator (400, 402) of claim 1, wherein the metal object is a bulge-shaped metal header block (502) positioned on one side of the resonator, the metal header block including an exterior surface (504) that faces away from the rest of the resonator, wherein the conductive material is applied to at least a portion of the exterior surface.

5. The resonator (400, 402) of claim 1, wherein the metal object is a metal disk (450) forming a back side of the resonator, the metal disk including an interior surface (454) that faces the coil element (304), wherein the conductive material is applied to at least a portion of the interior surface.

6. The resonator (400, 402) of claim 5, further comprising:

a metal ring (460) circumscribing the metal disk (450); and
a braze material coupling an outer diameter of the metal ring to the housing,

wherein the braze material is made of a material that facilitates further reducing the amount of heat induced during operation of the resonator.

7. The resonator (400, 402) of claim 6, wherein the conductive material is applied to at least a portion of the outer diameter of the metal ring (460).

8. A wireless power transfer system (200) comprising:

an external transmit resonator (202); and
an implantable receive resonator (204), the implantable receive resonator comprising:

a housing (420);
a magnetic core (418) positioned within the housing, the magnetic core defining an annular groove (310); and
a coil element (304) positioned within the annular groove,

**characterized in that** the implantable receive resonator further comprises a metal object, wherein a conductive material is applied to at least a portion of the metal object, wherein the conductive material facilitates reducing an amount of heat induced during operation of the implantable receive resonator.

9. A method of assembling a resonator (400, 402) for use in a wireless power transfer system (200), the method comprising:

positioning a magnetic core (410, 418) within a housing (412, 418), the magnetic core defining an annular groove (310); and
positioning a coil element (304) within the annular groove,
**characterized in that** the method further comprises applying a conductive material to at least a portion of a metal object of the resonator, wherein the conductive material facilitates reducing an amount of heat induced during operation of the resonator.

10. The method of claim 9, wherein the resonator (400, 402) is an implantable receive resonator.

11. The method of claim 9, wherein applying the conductive material to the metal object comprises applying silver, copper, gold and/or aluminum to at least a portion of the metal object.

12. The method of claim 9, wherein applying the conductive material to the metal object comprises applying a conducive material to at least a portion of a surface of a bulge-shaped metal header block (502) positioned on one side of the resonator (400, 402).

13. The method of claim 9, wherein applying the conductive material to the metal object comprises applying the conductive material to at least a portion of an interior surface of a metal disk (450) forming a back side of the resonator (400, 402).

14. The method of claim 13, further comprising:

coupling the metal disk (450) to a metal ring (460) circumscribing the metal disk; and
coupling an outer diameter of the metal ring to the housing (412, 420) using a braze material, wherein the braze material is made of a material that facilitates further reducing the amount of heat induced during operation of the resonator (400, 402).

15. The method of claim 14, further comprising applying a conductive material to at least a portion of the outer diameter of the metal ring (460).

FIG. 1

EP 4 668 540 A2

FIG. 2

FIG. 3

FIG. 4

EP 4 668 540 A2

FIG. 5

EP 4 668 540 A2

NO AG COAT

FIG. 6A

EP 4 668 540 A2

WITH 100 MICRON AG COAT

FIG. 6B

EP 4 668 540 A2